# EUROPÄISCHE PATENTSCHRIFT

(11) **EP 1 014 879 B1**
(45) Veröffentlichungstag und Bekanntmachung des Hinweises auf die Patenterteilung: **26.03.2003**
(21) Anmeldenummer: 99941436.0
(22) Anmeldetag: 16.07.1999
(51) Int. Cl.: A61B 17/88

(54) **SCHRAUBENDREHER FÜR MEDIZINISCHE ZWECKE**
MEDICAL SCREWDRIVER
TOURNEVIS MEDICAL

(30) Priorität: 17.07.1998 DE 19832303
(43) Veröffentlichungstag der Anmeldung: 05.07.2000
(73) Patentinhaber: Karl Storz GmbH & Co. KG, 78532 Tuttlingen (DE)
(72) Erfinder: STIHL, Ewald, D-78187 Geisingen (DE)
(74) Vertreter: Heuckeroth, Volker, Dr.
(86) Internationale Anmeldenummer: EP9905076
(87) Internationale Veröffentlichungsnummer: WO00003650

(56) Entgegenhaltungen:
- DE-C- 3 539 502
- DE-C- 3 714 994
- US-A- 2 302 691

## Beschreibung

Die Erfindung betrifft einen Schraubendreher zum Einbringen und Eindrehen einer Schraube im menschlichen oder tierischen Körper und/oder zum Ausdrehen und Entnehmen einer Schraube aus dem menschlichen Körper nach dem Oberbegriff des Anspruchs 1.

Ein derartiger Schraubendreher für medizinische Zwecke ist aus der DE 35 39 502 C1 bekannt.

Auf dem medizinisch chirurgischen Gebiet werden Schraubendreher verwendet, um Knochenschrauben im menschlichen oder tierischen Körper in Knochen einzudrehen bzw. wieder zu entfernen. Dort stellt sich häufig das Problem, daß das Operationsgebiet, in das die Schraube eingebracht werden soll, von außen nur schwer zugänglich ist, so daß es nicht möglich ist, die Schraube von Hand an den Zielort zu bringen und dort zu halten, um die Schraube dann gezielt und sicher einschrauben zu können, wenn nicht dazu eine große Öffnung im Körper geschaffen wird, um das Operationsgebiet freizulegen, oder eine zweite Inzision geschaffen wird, in die ein Instrument zum Führen und Halten der Schraube eingeführt werden kann.

Ein spezieller Anwendungsfall auf dem chirurgischen Gebiet ist die laparoskopische Wirbelsäulenoperation, bei der eine Knochenschraube oder nacheinander mehrere Knochenschrauben durch eine Operationsöffnung in der Bauchdecke durch die Bauchhöhle an die Wirbelsäule gebracht und in diese eingeschraubt werden. Für diese Operation ist ein Schraubendreher mit einem entsprechend langerstreckten Schaft erforderlich. Da es wünschenswert ist, eine solche Operation möglichst minimal-invasiv durchzuführen, d.h. von außen durch eine möglichst kleine Inzision in der Bauchdecke hindurch, besteht ein Bedarf an einem Schraubendreher, mit dem die Schraube nicht nur eingedreht, sondern zuvor durch die Bauchhöhle bis zur Wirbelsäule sicher eingeführt werden kann, ohne daß die Schraube beim Einführen von dem Schraubendreher abfällt. Auch muß die Schraube mit dem Schraubendreher so sicher gehalten werden können, daß sie am Zielort, an dem die Schraube eingedreht werden soll, angesetzt werden kann, ohne daß bei einer Druckausübung auf die Schraube diese seitlich verkippt. Außerdem sollte es möglich sein, die Schraube in einem Arbeitsgang sowohl an Ort und Stelle zu bringen als auch vollständig in den Knochen einzuschrauben, ohne daß die Festhalteeinrichtung dabei ein vollständiges Einschrauben der Schraube behindert.

Der aus der oben genannten DE 35 39 502 C1 bekannte Schraubendreher zum Einbringen und Eindrehen einer Knochenschraube weist einen Griff und einen Schaft auf, dessen vorderes Ende einen mit einem Schraubenkopf der Schraube zum Ein- bzw. Ausdrehen der Schraube in Eingriff bringbaren Kopf aufweist. Der Schraubendreher weist weiterhin eine Festhalteeinrichtung zum Festhalten der Schraube an dem Schaft auf, wobei die Festhalteeinrichtung einen am vorderen Ende einen in ihre Offenlage vorgespannte Spannzange aufweisenden, den Schaft umgebenden Innenrohrschaft und einen den Innenrohrschaft umgebenden Außenrohrschaft aufweist. Der Schraubendreher weist weiterhin eine Betätigungseinrichtung für den Innenrohrschaft und den Außenrohrschaft auf, die von einer ersten Stellung in eine zweite Stellung bewegbar ist, wodurch die Spannzange aus einer zurückgezogenen Stellung über den Kopf verschoben wird, und wobei die Betätigungseinrichtung aus der zweiten Stellung in eine dritte Stellung bewegbar ist, wodurch der Außenrohrschaft relativ zum Innenrohrschaft über die Spannzange verschoben wird, um diese zu schließen, und umgekehrt. Die Längsbewegung des Außenrohrschaftes bezüglich des Innenrohrschaftes ist in Richtung weg vom Werkzeug jeweils durch Anschläge begrenzt.

Bei diesem bekannten Schraubendreher sind der Innenrohrschaft, der Außenrohrschaft und der Schaft selbst vom Handgriff abnehmbar, wozu eine Kupplung zwischen dem Werkzeugschaft und dem Handgriff vorgesehen ist. Durch die Zerlegbarkeit des bekannten Schraubendrehers wird eine Sterilisierbarkeit des Schraubendrehers erzielt, so daß der bekannte Schraubendreher auch die Anforderungen an eine leichte Reinigbarkeit, die bei medizinischen Instrumenten stets gefordert wird, erreicht wird.

Nachteilig am Aufbau und an der Handhabbarkeit dieses bekannten Schraubendrehers ist allerdings, daß zum Abnehmen des Werkzeugschaftes eine von der Betätigungseinrichtung für die Festhalteeinrichtung separate Betätigungseinrichtung vorgesehen ist. Diese zweite Betätigungseinrichtung zum Zerlegen des Instruments umfaßt eine Schiebehülse, die zwischen der Betätigungseinrichtung für die Festhalteeinrichtung und dem Griff angeordnet ist. Die Handhabung des bekannten Schraubendrehers ist dadurch erschwert, daß die Betätigungseinrichtung für die Festhalteeinrichtung wegen der Betätigungseinrichtung zum Zerlegen des Instruments weit beabstandet ist, wodurch eine Einhandbedienung unmöglich oder zumindest erschwert ist.

Aus der DE 38 04 749 A1 ist weiterhin ein Schraubendreher mit einer Festhalteeinrichtung für Knochenschrauben bekannt. Bei diesem Schraubendreher ist die Möglichkeit einer Zerlegbarkeit in Griff und Schaftteile nicht erwähnt.

Aus der CH-PS 369 416 ist ebenfalls ein Schraubendreher mit einer Zange zum Festhalten einer Schraube bekannt, bei der die auf den Schraubenzieher schaftlose aufgeschobene Zangenhülse nach innen vorstehende, den ersteren angreifende und eine elastische Bremswirkung vermittelnde Haltemittel besitzt, derart, daß die Zangenhülse bei Verschiebung sich selbsttätig auf dem Schraubenzieherschaft hält und zwecks Freilegung des letzteren die Zangenhülse zufolge ihrer losen Anordnung ganz vom Schraubenzieherschaft abgezogen werden kann. Bei diesem bekannten Schraubendreher ist die Festhalteeinrichtung demnach nicht am Griff fest arretierbar.

Die US 2,370,407 offenbart einen Schraubendreher mit einer Festhalteeinrichtung für eine Schraube, die ebenfalls einen Innenrohrschaft mit Spannzange und einen Außenrohrschaft aufweist, der zum Schließen und Öffnen der Spannzange axial auf dem Innenrohrschaft verschiebbar ist. Als Betätigungseinrichtung zum Verschieben des Außenschaftes relativ zum Innenrohrschaft ist eine mit dem Außenrohrschaft fest verbundene und mit dem Innenrohrschaft in Gewindeeingriff stehende Mutter vorgesehen. Der Innenrohrschaft und der Außenrohrschaft sind im Betrieb des Schraubendrehers nicht am Handgriff arretiert.

Schließlich ist aus der DE 37 14 994 C1 ein Schraubendreher mit einer Schraubenhaltvorrichtung bekannt, bei der die Schraubenhaltevorrichtung als Teil auf ein Gewinde am Schaft eines Schraubenziehers aufgeschraubt werden kann. Dazu umfaßt die Schraubenhaltevorrichtung eine Haltebackenhülse, auf der die Hülse einer Rastermuffenhülse angeordnet ist. Die mit der Hülse verschraubbare Rastermuffe ist drehbar mit einer Einstellschraube verbunden. Für die Montage wird zunächst unter Zusammenpressung der Rasterzahnklammer die Haltebackenhülse von einer Seite in eine Schiebemuffe eingesetzt, nachdem zuvor die Hülse aufgeschoben wurde. Anschließend erfolgt von der anderen Seite die Einführung der Rastermuffe in die Schiebemuffe und deren Verschraubung mit der Hülse. Bei diesem bekannten Schraubendreher ist die Zerlegbarkeit konstruktiv aufwendig gelöst. Auch die Handhabung zum Zerlegen und Montieren des Schraubendrehers ist kompliziert.

Es ist daher eine Aufgabe der vorliegenden Erfindung, einen Schraubendreher der eingangs genannten Art dahingehend weiterzubilden, daß sowohl die Betätigung der Festhalteeinrichtung als auch das Zerlegen und Montieren des Schraubendrehers bei konstruktiv einfacher Gestaltung einfach zu handhaben ist.

Erfindungsgemäß wird diese Aufgabe hinsichtlich des eingangs genannten Schraubendrehers dadurch gelöst, daß die Betätigungseinrichtung am Griff angeordnet ist und in eine vierte Stellung bewegbar ist, in der der Innenrohrschaft und/oder Außenrohrschaft von dem Griff abnehmbar bzw. an diesen anbringbar ist/sind, wobei der Innenrohrschaft und/oder Außenrohrschaft durch Bewegen der Betätigungseinrichtung von der vierten Stellung in die erste Stellung an dem Griff arretiert bzw. umgekehrt entarretiert wird/werden.

Erfindungsgemäß lassen sich nicht nur die Funktionen Öffnen und Schließen der Spannzange sowie das Vorwärtsschieben bzw. Zurückziehen der Spannzange durch die am Griff vorgesehene Betätigungseinrichtung mit einer Hand bedienen, sondern auch das Zerlegen des Schraubendrehers in seine Einzelteile wird durch Bedienen derselben Betätigungseinrichtung bewerkstelligt, wodurch die Handhabung des erfindungsgemäßen Schraubendrehers vereinfacht wird. In der ersten, zweiten und dritten Stellung der Betätigungseinrichtung sind der Innenrohrschaft und der Außenrohrschaft stets fest mit dem Griff verbunden. Dadurch, daß die Betätigungseinrichtung zusätzlich eine vierte Zerlegestellung aufweist, wird es ermöglicht, daß die Betätigungseinrichtung für die Festhalteeinrichtung unmittelbar am Griff und dadurch bequem einhandbedienbar angeordnet werden kann, da keine weitere Betätigungseinrichtung für das Zerlegen erforderlich ist.

Somit wird die der Erfindung zugrunde liegende Aufgabe vollkommen gelöst.

In einer bevorzugten Ausgestaltung werden der Innenrohrschaft und der Außenrohrschaft durch Bewegung der Betätigungseinrichtung zwischen der ersten und der zweiten Stellung gemeinsam axial verschoben.

In einer weiteren bevorzugten Ausgestaltung ist/sind der Innenrohrschaft und/oder der Außenrohrschaft durch eine Schnellöseverbindung mit dem Griff verbunden.

Diese Maßnahme hat nun den weiteren Vorteil, daß die Handhabung des erfindungsgemäßen Schraubendrehers auch bei dessen Zerlegung wesentlich vereinfacht ist, und daß der Schraubendreher nach seiner Zerlegung auf einfach zu handhabende Weise wieder für einen erneuten Einsatz zusammengesetzt werden kann.

Dabei ist es bevorzugt, daß die Betätigungseinrichtung nur nach Entriegeln einer Sperre in die vierte Stellung bewegbar ist.

Diese Maßnahme hat den Vorteil, daß die Betätigungseinrichtung bei einem operativen Eingriff nicht versehentlich in die Zerlegestellung gebracht wird, wodurch die Betriebssicherheit des Schraubendrehers verbessert ist.

In weiteren bevorzugten Ausgestaltungen ist die Sperre mittels eines Druckknopfes oder durch Verdrehen der Beätigungseinrichtung entriegelbar.

Diese Maßnahmen stellen technisch einfache und leicht zu handhabende Ausgestaltungen der Sperre dar.

In einer weiteren bevorzugten Ausgestaltung rastet die Betätigungseinrichtung in der ersten, zweiten, dritten und/oder gegebenenfalls der vierten Stellung ein.

Diese Maßnahme hat insbesondere den Vorteil, daß der Operateur durch das Einrasten der Betätigungseinrichtung in der jeweiligen Stellung auch ohne Sichtkontrolle die Betriebsstellung der Spannzange, deren Schließ- bzw. deren Offenlage bzw. deren axiale Position wahrnehmen kann. Dies ist insbesondere beim Lösen der Spannzange von dem Schraubenkopf vor dem vollständigen Eindrehen der Schraube in den Knochen von Vorteil, wenn die Betätigungseinrichtung aus der dritten Stellung, in der die Schraube am Schraubendreher festgehalten ist, in die erste Stellung, in der die Spannzange hinter den Schraubenkopf zurückgezogen ist, so daß der Schraubenkopf vollständig freiliegt, bewegt wird. Ein weiterer Vorteil einer Einrastung in der dritten Stellung der Betätigungseinrichtung, in der die Spannzange geschlossen ist, besteht darin, daß auch bei losgelassener Betätigungseinrichtung diese in der dritten Stellung verbleibt, so daß sich die Spannzange nicht unerwünscht öffnet.

Dabei ist es bevorzugt, wenn die Betätigungseinrichtung für die erste, zweite, dritte und/oder gegebenenfalls vierte Stellung Kugelrasten aufweist.

Kugelrasten stellen eine technisch einfach zu realisierende und funktionssichere Möglichkeit von Rasten für die Betätigungseinrichtung dar. Darüber hinaus haben Kugelrasten den Vorteil, leichtgängig zu sein, so daß die Betätigungseinrichtung ohne großen Kraftaufwand und im wesentlichen ruckfrei aus den Raststellungen weiterbewegt werden kann.

In einer weiteren bevorzugten Ausgestaltung weist die Betätigungseinrichtung zumindest einen Schieber auf, der am Griff axial verschiebbar angeordnet ist.

Da die Funktion der Spannzange, nämlich Axialverschiebung und Öffnen und Schließen, durch Axialbewegungen des Innenrohrschaftes und des Außenrohrschaftes vermittelt werden, hat ein Schieber beispielsweise gegenüber einer drehbaren Betätigungseinrichtung den Vorteil, daß die Bewegungsübertragung von der Betätigungseinrichtung auf den Innenrohrschaft bzw. den Außenrohrschaft mechanisch einfach zu realisieren ist. Ein weiterer Vorteil besteht darin, daß mittels eines axialen Schiebers eine direkte Bewegungsübertragung auf den Innen- bzw. Außenrohrschaft ermöglicht wird, so daß die Bewegungsstrecke des Schiebers etwa gleich der Bewegungsstrecke von Innenrohrschaft und Außenrohrschaft ist, so daß sich der Operateur leichter an die Handhabung des erfindungsgemäßen Schraubendrehers gewöhnen kann.

Dabei ist es bevorzugt, wenn der Schieber am vorderen Ende des Griffs angeordnet ist.

Diese Maßnahme führt zu einem besonders bedienungsfreundlichen Schraubendreher, da der Operateur den Schieber der Betätigungseinrichtung beispielsweise mit dem Daumen und Zeigefinger derselben Hand, mit der er den Schraubendrehergriff hält, zum Betätigen der Spannzange bequem bedienen kann.

In einer weiteren bevorzugten Ausgestaltung weist die Betätigungseinrichtung zumindest ein erstes Steuerelement auf, das auf seiner Innenseite eine als Steuerkurve ausgebildete Nut aufweist, in die ein am hinteren Ende des Außenrohrschaftes angeordneter Stift eingreift, wobei das Steuerelement um eine quer zur Längsrichtung des Schaftes verlaufende Schwenkachse verschwenkbar am Griff gelagert ist, und wobei der Schieber einen Stift aufweist, der in eine Führungsnut auf der Außenseite des Steuerelementes eingreift, so daß durch axiales Verschieben des Schiebers das Steuerelement verschwenkt und dadurch der Außenrohrschaft verschoben wird.

Diese Ausgestaltung hat den Vorteil, daß eine axiale Verschiebung des Schiebers über das Steuerelement für die Funktionen Schließen und Öffnen der Spannzange in mechanisch einfacher und kinematisch günstiger Weise auf den Außenrohrschaft übertragen wird. Bei dieser Ausgestaltung kann die Verschiebung des Innenrohrschaftes dadurch bewerkstelligt werden, daß der Außenrohrschaft bei seiner axialen Verschiebung den Innenrohrschaft mitführt, wobei zum Schließen und Öffnen der Spannzange und die dafür vorgesehene Relativbewegung zwischen Außenrohrschaft und Innenrohrschaft ein Anschlag vorgesehen sein kann, durch den der Innenrohrschaft bei Erreichen seiner maximal vorderen Position stehenbleibt und nur noch der Außenrohrschaft axial weiter verschoben wird.

In einer weiteren bevorzugten Ausgestaltung weist die Betätigungseinrichtung zumindest ein zweites Steuerelement auf, das auf seiner Innenseite eine als Steuerkurve ausgebildete Nut aufweist, in die ein am hinteren Ende des Innenrohrschaftes angeordneter Stift eingreift, wobei das Steuerelement um eine quer zur Längsrichtung des Schafts verlaufende Schwenkachse verschwenkbar am Griff gelagert ist, und wobei der Schieber einen Stift aufweist, der in eine Führungsnut auf der Außenseite des Steuerelements eingreift, so daß durch axiales Verschieben des Schiebers das Steuerelement verschwenkt und dadurch der Innenrohrschaft verschoben wird.

Diese Maßnahme hat in Verbindung mit der vorherigen Ausgestaltung den Vorteil, daß für den Innenrohrschaft eine von der Steuerung des Außenrohrschaftes unabhängige Steuerung geschaffen wird, so daß gewährleistet ist, daß bei Betätigung der Betätigungseinrichtung die Funktionen Schließen und Öffnen der Spannzange sowie Zurückziehen und Vorwärtsschieben der Spannzange störungsfrei ausgeführt werden.

Dabei ist bevorzugt, wenn die Nut des ersten Steuerelements bzw. die Nut des zweiten Steuerelements an ihrem vorderen Ende offen ist, und daß der Stift des Außenrohrschaftes bzw. der Stift des Innenrohrschaftes axial in die Nut einfahren und aus dieser ausfahren kann, wenn die Betätigungseinrichtung in ihrer vierten Stellung ist, und daß der Stift des Innenrohrschaftes bzw. der Stift des Außenrohrschaftes in der Nut gefangen ist, sobald die Betätigungseinrichtung in die erste Stellung bewegt worden ist.

Diese Maßnahme hat den weiteren Vorteil, daß der Innenrohrschaft bzw. der Außenrohrschaft durch Zusammenschieben mit dem Griff selbsttätig mit der Betätigungseinrichtung in Eingriff kommen und durch eine leicht zu handhabende Bewegung der Betätigungseinrichtung an dem Griff arretiert bzw. wieder entarretiert werden können. Mit dieser Ausgestaltung wird eine mechanisch vorteilhaft einfache Schnellöseverbindung zwischen dem Innenrohrschaft bzw. dem Außenrohrschaft einerseits und dem Griff andererseits geschaffen.

In einer weiteren bevorzugten Ausgestaltung weist eine distale Stirnseite des Schiebers eine nicht runde Öffnung auf, durch die der Außenrohrschaft durchgeführt ist, wobei ein hinterer Endabschnitt des Außenrohrschaftes eine entsprechende komplementäre Umfangsgestalt aufweist.

Diese Maßnahme hat den Vorteil, daß der Außenrohrschaft, in dem der Innenrohrschaft aufgenommen ist, nur in einer vorbestimmten Drehlage relativ zu dem Griff mit diesem verbunden werden kann, so daß beim Einschieben des Außenrohrschaftes in den Schieber der Außenrohrschaft automatisch die zum Verbinden des Außenrohrschaftes mit dem Griff bzw. mit den vorgenannten Steuerelementen richtige Drehorientierung aufweist.

In einer weiteren bevorzugten Ausgestaltung weist die Betätigungseinrichtung zwei Schieber auf, die am Griff axial verschiebbar angeordnet sind, wobei der eine Schieber mit dem Außenrohrschaft und der andere Schieber mit dem Innenrohrschaft verbunden ist.

Auch diese Maßnahme ist vorteilhaft, da mit zwei Schiebern an Stelle eines Schiebers, wobei der eine Schieber zur Betätigung des Außenrohrschaftes und der andere Schieber zur Betätigung des Innenrohrschaftes dient, eine direkte Übertragung der axialen Bewegung des jeweiligen Schiebers auf den Außenrohrschaft bzw. den Innenrohrschaft ermöglicht wird.

Dabei ist es weiterhin bevorzugt, wenn der eine Schieber zwischen der ersten oder zweiten Stellung bewegbar ist, um die geöffnete Spannzange axial zu verschieben, und daß der andere Schieber zwischen der zweiten und der dritten Stellung bewegbar ist, um den Außenrohrschaft relativ zu dem Innenrohrschaft zu verschieben, um die Spannzange zu schließen bzw. zu öffnen.

Alternativ dazu ist es bevorzugt, wenn die Betätigungseinrichtung ein am Griff angeordnetes drehbares Griffelement aufweist, das in die erste, zweite, dritte und gegebenenfalls vierte Stellung gedreht werden kann.

Auch diese Ausgestaltung der Betätigungseinrichtung führt zu einer vorteilhaft einfachen Handhabung des erfindungsgemäßen Schraubendrehers in Ein-Hand-Bedienung.

Dabei ist es weiterhin bevorzugt, wenn das drehbare Griffelement innenliegende Steuerkurven aufweist, in die am Außenrohrschaft und am Innenrohrschaft vorgesehene Führungsstifte eingreifen, wobei die Steuerkurven derart ausgebildet sind, daß eine Drehung des Griffelementes in eine axiale Bewegung des Innenrohrschaftes und des Außenrohrschaftes sowie eine Relativverschiebung zwischen dem Innenrohrschaft und dem Außenrohrschaft übertragen wird.

Bei dieser Maßnahme besteht der Vorteil darin, daß die Steuerkurven unmittelbar in dem drehbaren Griffelement ausgebildet werden können, ohne daß dazu weitere Teile erforderlich sind. An Stelle der zuvor beschriebenen Betätigungseinrichtungen, die einen axial beweglichen oder zwei axial bewegliche Schieber aufweisen oder ein drehbares Griffelement, sind auch Betätigungseinrichtungen für den erfindungsgemäßen Schraubendreher bevorzugt, die zwischen zwei oder mehr Stellungen der Betätigungseinrichtung verschiebbar und zwischen den übrigen Stellungen verdrehbar sind. Beispielsweise kann eine Betätigungseinrichtung vorgesehen sein, die zwischen der ersten und zweiten Stellung axial bewegbar ist, und von der zweiten in die dritte Stellung durch eine Drehbewegung betätigt wird.

In einer weiteren bevorzugten Ausgestaltung ist der Innenrohrschaft zumindest in der dritten Stellung relativ zu dem Außenrohrschaft verdrehbar.

Diese Maßnahme hat den weiteren Vorteil, daß beim Ein- bzw. Ausdrehen der Schraube in den Knochen der Außenrohrschaft in dem Körper nicht gedreht werden muß, wodurch beim Drehen des Schraubendrehers an dem Außenrohrschaft anliegendes Gewebe im menschlichen Körper nicht traumatisiert wird.

In einer weiteren bevorzugten Ausgestaltung ist der Kopf des Schafts auswechselbar.

Diese Maßnahme hat den Vorteil, daß ein und derselbe Schraubendreher für verschiedene Schraubentypen mit unterschiedlichen Schraubenköpfen benutzt werden kann.

Weitere Vorteile ergeben sich aus der nachfolgenden Beschreibung und der beigefügten Zeichnung.

Ein Ausführungsbeispiel der Erfindung ist in der Zeichnung dargestellt und wird hiernach mit Bezug auf die Figuren näher beschrieben. Es zeigen:
- Fig. 1: einen Schraubendreher in einer Gesamtdarstellung, teilweise im Längsschnitt entlang einer Längsmittelebene des Schraubendrehers;
- Fig. 2: den Innenrohrschaft mit Spannzange im von dem Griff des Schraubendrehers abgenommenen Zustand, teilweise im Längsschnitt;
- Fig. 3: den Außenrohrschaft des Schraubendrehers im von dem Griff des Schraubendrehers abgenommenen Zustand;
- Fig. 4: einen Querschnitt durch den Außenrohrschaft entlang der Linie IV-IV in Fig. 3;
- Fig. 5: einen Längsschnitt entlang einer Längsmittelebene des Handgriffs des Schraubendrehers bei abgenommenem Außenrohrschaft und Innenrohrschaft;
- Fig. 6: einen Schnitt entlang der Linie VI-VI in Fig. 5;
- Fig. 7: eine Vorderansicht des Schiebers der Betätigungseinrichtung des Schraubendrehers;
- Fig. 8: eine Seitenansicht auf eine Außenseite eines Steuerelementes der Betätigungseinrichtung;
- Fig. 9: eine Seitenansicht auf eine Innenseite des Steuerelementes in Fig. 8;
- Fig. 10: eine der Fig. 9 entsprechende Seitenansicht auf ein weiteres Steuerelement der Betätigungseinrichtung; und
- Fig. 11 a) bis 11 d): eine schematische Darstellung der Funktion des Schraubendrehers in vier Teilbildern.

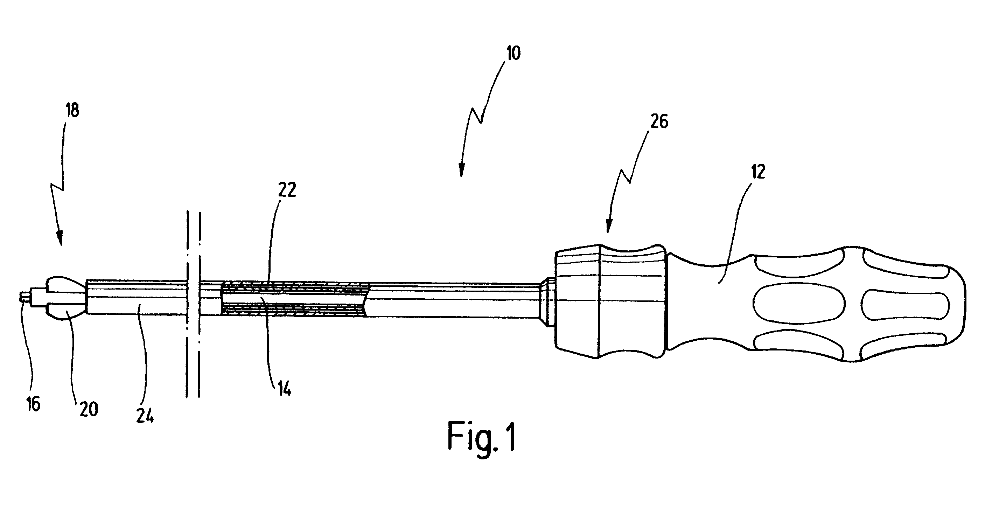

In Fig. 1 ist ein mit dem allgemeinen Bezugszeichen 10 versehener Schraubendreher dargestellt. Der Schraubendreher 10 dient zum Einbringen und Eindrehen einer Schraube im menschlichen und tierischen Körper und/oder zum Ausdrehen und Entnehmen einer Schraube aus dem menschlichen oder tierischen Körper.

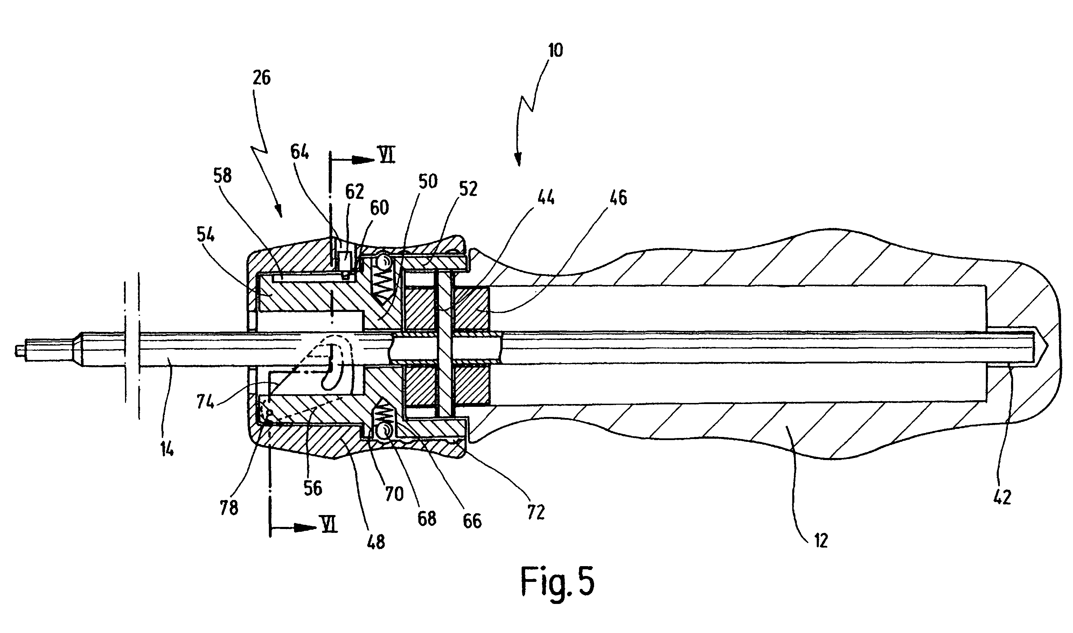

Der Schraubendreher 10 weist an seinem hinteren Ende einen Griff 12 auf. Mit dem Griff 12 ist, wie aus Fig. 5 hervorgeht, ein Schaft 14 drehfest verbunden, wobei der Schaft 14 an seinem distalen Ende einen mit einem Schraubenkopf einer Schraube zum Ein- bzw. Ausdrehen der Schraube in Eingriff bringbaren Kopf 16 aufweist. Der Kopf 16 ist an dem Schaft 14 auswechselbar angebracht. Der Schaft 14 ist lang erstreckt und weist eine Länge von etwa 40 bis 50 cm auf.

Der Schraubendreher 10 weist weiterhin eine Festhalteeinrichtung 18 zum Festhalten der Schraube an dem Kopf 16 auf. Die Festhalteeinrichtung 18 weist eine Spannzange 20 auf, die in Fig. 1 in ihrer Offenstellung, in die die Spannzange 20 vorgespannt ist, dargestellt ist.

Die Spannzange 20 sitzt am vorderen Ende eines Innenrohrschaftes 22, der um den Schaft 14 herum angeordnet und auf diesem axial verschiebbar ist. Der Innenrohrschaft 22 erstreckt sich etwa bis zum Handgriff 12, wie noch näher beschrieben werden wird.

Die Festhalteeinrichtung 18 weist ferner einen Außenrohrschaft 24 auf, der um den Innenrohrschaft 22 herum angeordnet ist, und der sowohl relativ zu dem Innenrohrschaft 22 als auch gemeinsam mit diesem axial verschiebbar ist.

Ferner weist der Schraubendreher 10 eine Betätigungseinrichtung 26für den Innenrohrschaft 22 und den Außenrohrschaft 24 auf, die am vorderen Ende des Griffes 12 angeordnet ist. Die Betätigungseinrichtung 26 wird ebenfalls später noch näher beschrieben.

Der Innenrohrschaft 22 und der Außenrohrschaft 24 sind von dem Griff 12 abnehmbar.

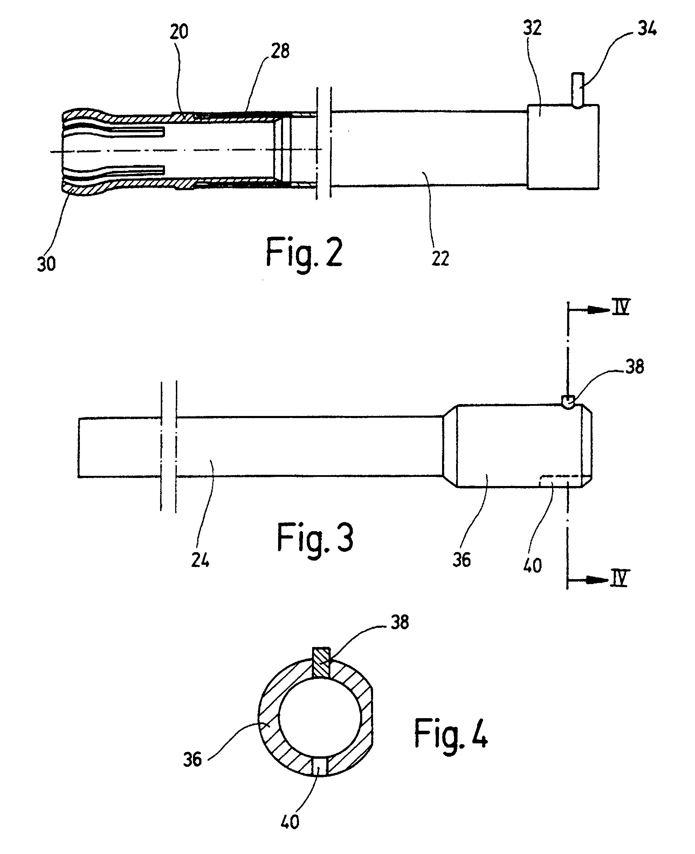

In Fig. 2 ist der Innenrohrschaft 22 in Alleinstellung dargestellt. Die am vorderen Ende des Innenrohrschaftes 22 angeordnete Spannzange 20 ist über eine hintere Hülse 28 fest mit dem Innenrohrschaft 22 verbunden. An ihrem vorderen Ende weist die Spannzange 20 vier durch axiale Einschnitte voneinander getrennte, umfänglich achsensymmetrisch angeordnete Zungen 30 auf, die radial elastisch zusammendrückbar sind, und die ohne äußere Krafteinwirkung selbsttätig eine radial gespreizte Lage einnehmen. Die Spannzange 20 ist somit in ihre Offenlage vorgespannt.

An einem radial verbreiterten Endabschnitt 32 des Innenrohrschaftes 22 ist ein radial nach außen stehender Stift 34 fest mit dem Endabschnitt 32 verbunden.

In Figuren 3 und 4 ist der Außenrohrschaft 24 in von dem Griff 12 abgenommenen Zustand in Alleinstellung dargestellt. Ein hinterer Endabschnitt 36 des Außenrohrschaftes 24 ist radial verbreitert. Mit dem Endabschnitt 36 ist ein radialer Stift 38 fest verbunden. Dem Stift 38 diametral gegenüber liegend ist in dem Endabschnitt 36 eine axial oblong ausgebildete Aussparung 40 vorgesehen, die dazu dient, den Stift 34 des Innenrohrschaftes 22 aufzunehmen, wenn der Außenrohrschaft 24 auf den Innenrohrschaft 22 aufgeschoben ist. Der Innenrohrschaft 22 läßt sich mit der Spannzange 20 voran in das hintere Ende des Außenrohrschaftes 24 einschieben, bis der Stift vollkommen in die Ausnehmung 40 eingefahren ist und an das vordere Ende derselben anschlägt. Die Länge des Innenrohrschaftes 22 und des Außenrohrschaftes 24 sind so aufeinander abgestimmt, daß im aufeinandergeschobenen Zustand von Innenrohrschaft 22 und Außenrohrschaft 24 die Zungen 30 aus dem vorderen Ende des Außenrohrschaftes 24 gerade soweit vorragen, daß sie ihre Offenstellung einnehmen können. Der Außendurchmesser der Gesamtheit der Zungen 30 in deren Offenlage ist geringfügig größer als der Innendurchmesser des Außenrohrschaftes 24. Wird der Außenrohrschaft 24 über die Zungen 30 geschoben, werden diese somit zusammengedrückt, wodurch die Spannzange 20 geschlossen wird.

In Figuren 5 bis 7 ist der Schraubendreher 10 bei abgenommenem Innenrohrschaft 22 und abgenommenen Außenrohrschaft 24 dargestellt. Wie aus Fig. 5 hervorgeht, erstreckt sich der Schaft 14 bis zum hinteren Ende des Griffes 12, wo das hintere Ende des Schaftes 14 achsenzentriert in einem Sackloch 42 des Griffes 12 aufgenommen und fest mit dem Griff 12 verbunden ist. Ein diametral durch den Schaft 14 durchgehender Verdrehsicherungsstift 44 sorgt für eine axial und drehfeste Verbindung des Schaftes 14 mit dem Griff 12. Der Verdrehsicherungsstift 44 ist dabei zusätzlich durch einen stabilen drehfesten Zentrierring 46 durchgeführt.

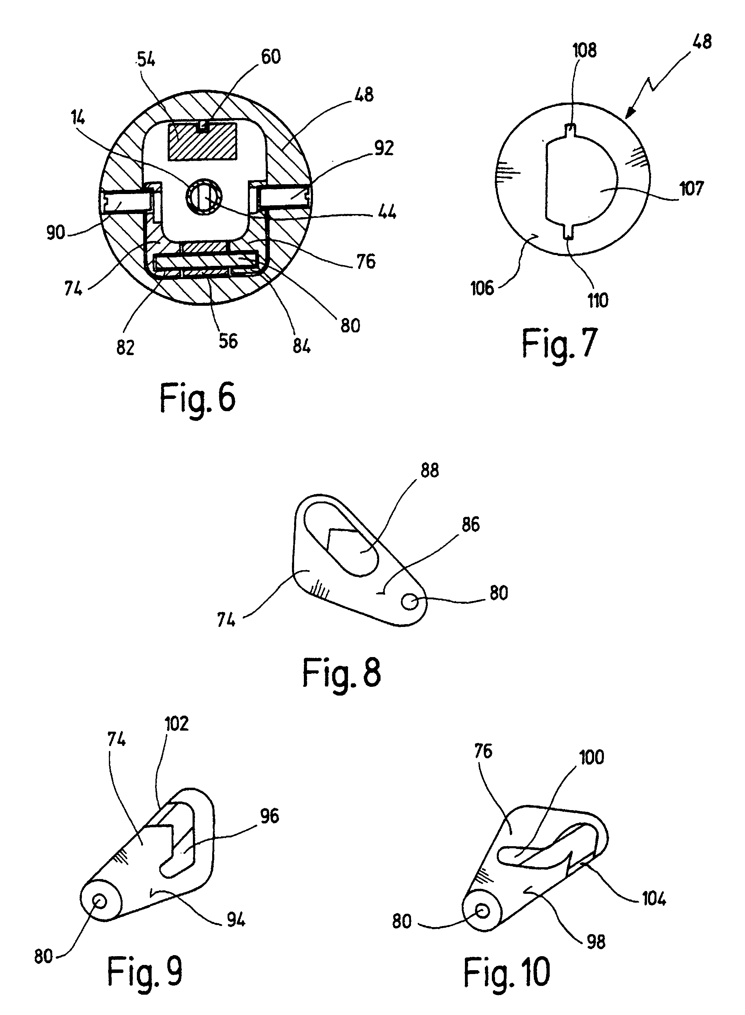

Die Betätigungseinrichtung 26 weist einen Schieber 48 auf, der am vorderen Ende des Griffes 12 angeordnet, und in Form einer Hülse ausgebildet ist. Der Schieber 48 ist dazu axial verschiebbar, jedoch unverdrehbar auf einem Lagerelement 50 gelagert, das über einen hülsenartigen proximalen Endabschnitt 52 fest mit dem Griff 12 verbunden ist. An seinem vorderen Ende weist das Lagerelement 50 einen sich axial erstreckenden ersten (in Figuren 5 und 6 oberen) Steg 54 und einen sich ebenfalls axial erstreckenden zweiten (in Figuren 5 und 6 unteren) Steg 56 auf.

In dem ersten Steg 54 ist eine Führungsnut 58 ausgebildet, in der eine radiale stumpfe Spitze 60 einer Schraube 62, die in eine radiale Öffnung 64 in dem Schieber 48 eingeschraubt ist, geführt wird. Die Führungsnut 58 ist umfänglich begrenzt, so daß der Schieber 48 auf dem Lagerelement 50 axial verschoben, aber nicht verdreht werden kann. Es kann auch vorgesehen sein, daß die Führungsnut nicht axial gerade ausgebildet ist, sondern einen Z-förmigen Verlauf aufweist, so daß der Schieber 48 über einen ersten Abschnitt der Führungsnut 58 nur axial verschiebbar, über einen zweiten Abschnitt der Führungsnut 58 nur verdrehbar und über einen dritten Abschnitt der Führungsnut 58 wiederum nur axial verschiebbar ist. Dies kann dazu ausgenutzt werden, eine Sperre für den Schieber 48 zu schaffen, wie später noch näher erläutert wird.

In dem mittleren Abschnitt des Lagerelementes 50 sind umfänglich verteilt radial einschneidende Sacklöcher 66 vorgesehen, in denen jeweils eine Kugel 68 aufgenommen ist, die mittels einer Feder 70 radial nach außen gedrückt wird. Zusammen mit kugelkalottenförmigen Ausnehmungen 72 auf der Innenseite des Schieber 48 bilden die Kugeln 68 Kugelrasten, wobei bei dem gezeigten Ausführungsbeispiel drei axiale Raststellungen des Schiebers 48 gemäß drei axial beabstandeten kalottenförmigen Ausnehmungen 72 vorgesehen sind, die im Zusammenhang mit der Funktion des Schaubendrehers 10 bestimmten Stellungen der Betätigungseinrichtung 26 entsprechen, wie hiernach noch näher erläutert wird.

Die Betätigungseinrichtung 26 weist weiterhin ein erstes Steuerelement 74 zur Steuerung der Axialbewegung des Außenrohrschaftes 24 auf, das in Fig. 5 teilweise mit unterbrochenen Linien dargestellt ist, da das erste Steuerelement 74, wie aus Fig. 6 hervorgeht, seitlich der Längsmittelachse des Schraubendrehers 10 positioniert ist. Weiterhin ist ein zweites Steuerelement 76 zur Steuerung der axialen Bewegung des Innenrohrschaftes 22 vorgesehen.

Das erste Steuerelement 74 und das zweite Steuerelement 76 sind um eine gemeinsame Schwenkachse 78 gemeinsam verschwenkbar an dem zweiten Steg 56 gelagert, wobei die Schwenkachse 78 durch einen Lagerzapfen 80 gebildet wird, der durch den zweiten Steg 56 beidseitig durchgeht und in ein Sackloch 82 des ersten Steuerelementes 74 bzw. ein Sackloch 84 des zweiten Steuerelementes 76 eingreift und darin fest mit dem ersten Steuerelement 74 und dem zweiten Steuerelement 76 verbunden ist.

Das erste Steuerelement 74 ist in vergrößertem Maßstab in Alleinstellung in Figuren 8 und 9 dargestellt, während das zweite Steuerelement 76 in Alleinstellung in Fig. 10 dargestellt ist.

Gemäß Fig. 8 ist auf einer Außenseite 86 des ersten Steuerelementes 74 eine oblong ausgebildete Ausnehmung 88 ausgespart, in die gemäß Fig. 6 ein in den Schieber 48 eingeschraubter Stift 90 eingreift. Entsprechend ist auf einer nicht dargestellten Außenseite des zweiten Steuerelementes 76 eine der Ausnehmung 88 entsprechende Ausnehmung vorgesehen, in die ein Stift 92, der in den Schieber 48 dem Stift 90 gegenüberliegend eingeschraubt ist, eingreift. Die Stifte 90 und 92 bewirken bei einer axialen Verschiebung des Schiebers 48 auf dem Lagerelement 50 eine Verschwenkung des ersten Steuerelementes 74 und des zweiten Steuerelementes 76 um die Schwenkachse 78.

Auf einer Innenseite 94 des ersten Steuerelementes 74 ist eine Steuerkurve in Form einer Nut 96 ausgebildet (vergl. Fig. 9). Auf einer Innenseite 98 des zweiten Steuerelementes 76 ist eine Steuerkurve in Form einer Nut 100 ausgebildet (vergl. Fig. 10), wobei sich die Steuerkurve gemäß der Nut 100 von der Steuerkurve gemäß der Nut 96 bezüglich des Kurvenverlaufes unterscheidet.

Das Steuerelement 74 mit der Nut 96 dient der Steuerung der axialen Bewegung des Außenrohrschaftes 24, wobei der Stift 38 (vergl. Figur 6) des Außenrohrschaftes 24 im montierten Zustand in die Nut 96 eingreift. Entsprechend dient das Steuerelement 76 mit der Nut 100 der Steuerung der axialen Bewegung des Innenrohrschaftes 22, wobei der Stift 34 des Innenrohrschaftes 22 (vergl. Fig. 2) im montierten Zustand in die Nut 100 eingreift.

Die Nut 96 weist weiterhin ein offenes Ende 102 und die Nut 100 weist ein offenes Ende 104 auf, die dem Einführen der Stifte 34 und 38 in die Nut 96 bzw. 100 beim Zusammensetzen von Innenrohrschaft 22 und Außenrohrschaft 24 mit dem Griff dienen.

Wie in Fig. 7 dargestellt ist, weist eine distale Stirnseite 106 des Schiebers 48 eine dem Endbschnitt 36 des Außenrohrschaftes 24 angepaßte nicht runde Öffnung 107 auf, wobei in der Stirnseite 106 zusätzlich diametral gegenüberliegende radiale Aussparungen 108 und 110 vorgesehen sind, durch die die Stifte 34 und 38 des Innenrohrschaftes 22 bzw. des Außenrohrschaftes 24 durchgeführt werden können. Durch die Gestaltung der Öffnung 107 der Stirnseite 106 und des Endabschnittes 36 des Außenrohrschaftes 24 läßt sich der Außenrohrschaft 24 nur in der durch die Öffnung der Stirnseite 106 vorgegebenen Drehlage in den Schieber 48 zum Verbinden des Außenrohrschaftes und des Innenrohrschaftes mit dem Griff 12 einführen.

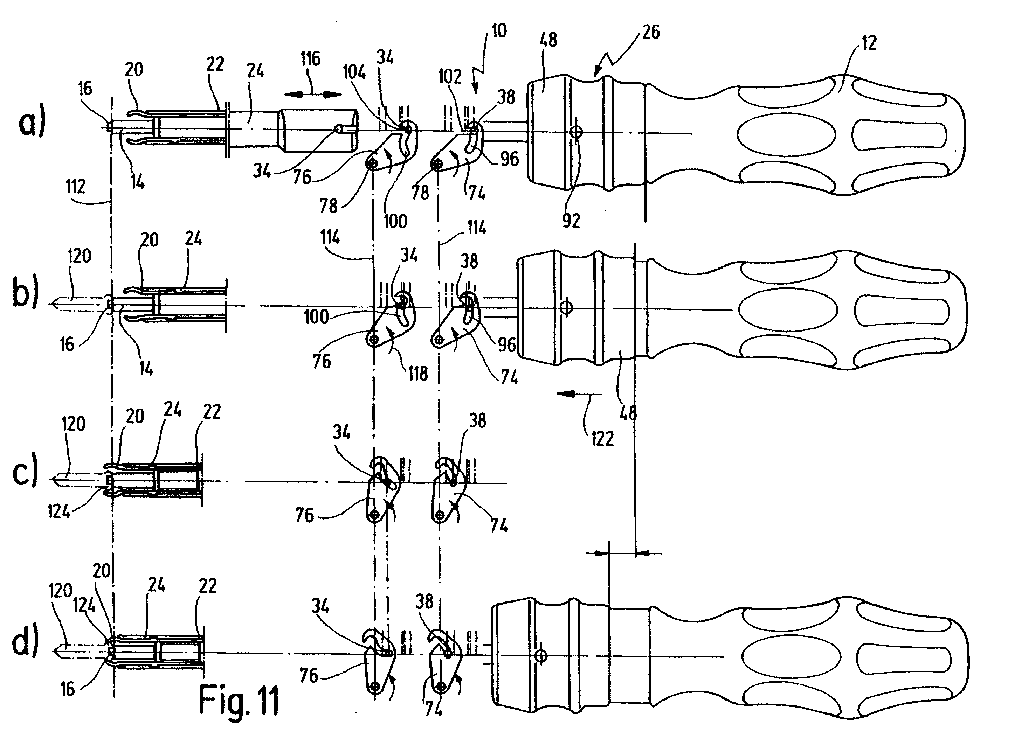

Mit Bezug auf Fig. 11 wird hiernach die Montage bzw. Zerlegung des Schraubendrehers 10 sowie die Funktion des Schraubendrehers 10 näher beschrieben. Zur Veranschaulichung der Bewegungsabläufe wurden das erste Steuerelement 74 und das zweite Steuerelement 76 von der Betätigungseinrichtung 26 losgelöst dargestellt, wobei zur weiteren Veranschaulichung das erste Betätigungselement 74 und das zweite Betätigungselement 76 axial voneinander beabstandet dargestellt sind. Es versteht sich jedoch, daß das erste Steuerelement 74 und das zweite Steuerelement 76 axial auf gleicher Höhe und gemäß der in Fig. 5 und Fig. 6 gezeigten Anordnung an dem zweiten Steg 56 des Lagerelementes 50 befestigt sind.

In Figuren 11 a) bis 11 d) sind als axial ortsfeste Bezugslinien eine strichpunktierte Linie 112 eingezeichnet, die der axialen Position der unbeweglichen Klinge 16 anzeigt, sowie als weitere axial ortsfeste Bezugslinie eine strichpunktierte Linie 114, die aufgrund der besonderen axial beabstandeten Darstellung des ersten Steuerelementes 74 und des zweiten Steuerelementes 76 doppelt eingezeichnet ist, und die die axial ortsfeste Lage der Schwenkachse 78 des ersten Steuerelementes 74 bzw. des zweiten Steuerelementes 76 wiedergibt.

Die Betätigungseinrichtung 26 bzw. der Schieber 48 weist vier Stellungen auf, die in Figuren 11 a) bis 11 d) veranschaulicht sind.

In Fig. 11 a) ist der Schieber 48 in der vierten Stellung dargestellt, in der der Schieber 48 in seine maximal proximale Lage zurückgezogen ist. In dieser Stellung der Betätigungseinrichtung 26 lassen sich der Innenrohrschaft 22 und der Außenrohrschaft 24 mit dem Griff 12 verbinden bzw. von diesem abnehmen, wie mit einem Doppelpfeil 116 angedeutet ist. In dieser vierten Stellung des Schiebers 48, die auch in Fig. 5 dargestellt ist, sind das erste Steuerelement 74 und das zweite Steuerelement 76 um die Schwenkachse 78 nach proximal verschwenkt, so daß das offene Ende 102 der Nut 96 des ersten Steuerelementes 74 und das offene Ende 104 der Nut 100 des zweiten Steuerelementes 76 in Richtung distales Ende des Schraubendrehers 10 zeigen. In dieser Stellung können der Stift 34 des Innenrohrschaftes 22 und der Stift 38 des Außenrohrschaftes 24 in die offenen Enden 102 bzw. 104 ein- bzw. ausfahren. Für diese vierte Stellung, d.h. die Zerlegestellung der Betätigungseinrichtung 26 ist gemäß Fig. 5 eine Raststellung des Schiebers 48 vorgesehen.

Das Einfahren der Stifte 34 und 38 erfolgt automatisch durch Einschieben des Innenrohrschaftes 22 und des Außenrohrschaftes 24 in den Schieber 48, bis ein Anschlagen verspürt wird.

Ausgehend von der vierten Stellung der Betätigungseinrichtung 26 wird der Schieber 48 anschließend axial in die erste Stellung verschoben, die in Fig. 11 b) dargestellt ist. Durch das axiale Verschieben des Schiebers 48 aus der vierten Stellung in die erste Stellung werden das erste Steuerelement 74 und das zweite Steuerelement 76 um ihre gemeinsame Schwenkachse 78 in Richtung eines Pfeiles 118 um einen kleinen Winkel verschwenkt. Der Stift 34 des Innenrohrschaftes 22 und der Stift 38 des Außenrohrschaftes 24 sind nun in dieser ersten Stellung der Betätigungseinrichtung 26 in der Nut 100 bzw. der Nut 96 gefangen, so daß der Innenrohrschaft 22 und der Außenrohrschaft 24 in dieser ersten Stellung nicht mehr axial von dem Griff 12 abgezogen werden können, sondern fest mit diesem verbunden sind.

Für die erste Stellung der Betätigungseinrichtung 26 ist gemäß Fig. 5 ebenfalls eine Raststellung an dem Schieber 48 vorgesehen. Bei der Bewegung des Schiebers 48 aus der vierten Stellung in die erste Stellung führen der Innenrohrschaft 22 und der Außenrohrschaft 24 eine geringfügige axiale Bewegung aus, wie sich aus einem Vergleich der axialen Lage der Stifte 34 und 38 in Fig. 11 a) und Fig. 11 b) ergibt.

In der ersten Stellung der Betätigungseinrichtung 26 kann nun auf den Kopf 16 des Schaftes 14 eine Schraube 120 aufgesteckt werden. Die Spannzange 20 befindet sich trotz der vorerwähnten geringfügigen axialen Bewegung des Innenrohrschaftes 22 und des Außenrohrschaftes 24 noch in einer gegenüber dem Kopf 16 zurückgezogenen Stellung, in der die Spannzange 20 das Aufstecken der Schraube 120 nicht behindert.

Durch weiteres Schieben des Schiebers 48 in Richtung eines Pfeiles 122 in eine zweite Stellung werden das erste Steuerelement 74 und das zweite Steuerelement 76 weiter in Richtung des Pfeiles 118 verschwenkt, bis sie ihre in Fig. 11 c) dargestellte Drehlage erreicht haben. Durch die Verschwenkung des ersten Steuerelementes 74 und des zweiten Steuerelementes 76 und aufgrund der Ausgestaltung der Nut 100 bzw. der Nut 96 werden der Stift 34 des Innenrohrschaftes 22 und der Stift 38 des Außenrohrschaftes 24 gemeinsam axial nach distal verschoben, so daß der Außenrohrschaft 24 und der Innenrohrschaft 22 gemeinsam axial bewegt werden. Dabei wird die Spannzange 20 in geöffnetem Zustand über einen Schraubenkopf 124 der Schraube 120 geschoben, wie in Fig. 11 c) dargestellt ist.

Für die zweite Stellung des Schiebers 48 ist in dem gezeigten Ausführungsbeispiel keine Raststellung des Schiebers 48 vorgesehen.

Durch weiteres axiales Verschieben des Schiebers 48 in Richtung des Pfeiles 122 aus der zweiten Stellung in Fig. 11 c) in die in Fig. 11 d) gezeigte dritte Stellung wird nun der Außenrohrschaft 24 relativ zu dem Innenrohrschaft 22 verschoben, wodurch sich das distale Ende des Außenrohrschaftes 24 über die Spannzange 20 schiebt und diese in ihre Schließlage zusammendrückt, wodurch der Schraubenkopf 124 der Schraube 120 von der Spannzange 20 umfaßt und somit die Schraube 120 an dem Kopf 16 unverlierbar und in axialer Ausrichtung mit der Längsachse des Schraubendrehers festgehalten wird.

Die Relativbewegung zwischen dem Außenrohrschaft 24 und dem Innenrohrschaft 22 wird dadurch erreicht, daß die Nut 100 des zweiten Steuerelementes 76, das der Steuerung des Innenrohrschaftes 22 dient, so ausgebildet ist, daß das zweite Steuerelement 76 bei der Verschwenkung aus der in Fig. 11 c) dargestellten Drehlage in die in Fig. 11 d) dargestellte Drehlage den in der Nut 100 aufgenommenen Stift 34 des Innenrohrschaftes 22 nicht axial verschiebt. Die Nut 96 des ersten Steuerelementes 74, das der Steuerung des Außenrohrschaftes 24 dient, ist im Unterschied dazu so ausgebildet, daß bei der Verschwenkung des ersten Steuerelementes 74 aus der in Fig. 11 c) dargestellten Drehlage in die in Fig. 11 d) dargestellte Drehlage der Stift 38 axial nach distal bewegt wird, woraus sich die Relativverschiebung zwischen dem Außenrohrschaft 24 und dem Innenrohrschaft 22 ergibt.

Für die dritte, in Fig. 11 d) dargestellte Stellung des Schiebers 48 ist wiederum eine Raststellung des Schiebers 48 auf dem Lagerelement 50 vorgesehen, wodurch die Spannzange 20 in ihrer Schließlage selbsthaltend arretiert ist.

Die nunmehr an dem Kopf 16 festgehaltene Schraube 120 kann mittels des Schraubendrehers 10 durch eine Inzision in den Körper eingebracht und an den gewünschten Zielort, beispielsweise an der Wirbelsäule, an dem die Schraube 120 eingedreht werden soll, angesetzt werden. Die Schraube 120 kann nun durch Drehen des Griffes 12 einige Gewindegänge in den Knochen eingeschraubt werden, bis die Schraube 120 in dem Knochen eine ausreichende Selbstfixierung erfahren hat. Zum vollkommenen Eindrehen der Schraube 120 in den Knochen wird dann der Schieber 48 umgekehrt aus der in Fig. 11 c) dargestellten Lage in die in Fig. 11 b) dargestellte erste Stellung zurückgezogen, wodurch sich die Spannzange 20 zunächst öffnet und dann hinter den Schraubenkopf 124 zurückgezogen wird. Die Schraube 120 kann nunmehr vollends in den Knochen eingeschraubt werden, bis der Kopf 124 der Schraube 120 versenkt ist.

Es versteht sich, daß der Schraubendreher 10 entsprechend zum Ausdrehen und zum Entnehmen einer Schraube aus dem menschlichen oder tierischen Körper verwendet werden kann, wozu dann die Betätigungseinrichtung 26 entsprechend bedient wird, ohne daß dies hier einer näheren Erläuterung bedarf.

Der Schieber 48 eignet sich insbesondere für eine Einhand-Bedienung, in dem der Schieber 48 beispielsweise mit dem Daumen und dem Zeigefinger derselben Hand, mit der der Griff 12 gehalten wird, bedient wird.

Um die Betriebssicherheit des Schraubendrehers 10 zu erhöhen, kann zusätzlich vorgesehen sein, daß die Betätigungseinrichtung 26 von der in Fig. 11 b) dargestellten ersten Stellung in die in Fig. 11 a) dargestellte ersten Stellung nur dann bewegt werden kann, wenn zuvor von der Bedienungsperson eine Sperre gelöst wurde. Eine solche Sperre kann in Form einer mittels eines Druckknopfes auslösbaren Sperre ausgebildet sein, oder, wie bereits an einer vorherigen Stelle beschrieben wurde, indem die Führungsnut 58 des Lagerelementes 50 eine Z-förmige Ausgestaltung aufweist, so daß der Schieber 48 nur dann von der ersten Stellung in die vierte Stellung bewegt werden kann, wenn der Schieber 48 vorher verdreht wird.

Weiterhin kann bei dem Schraubendreher 10 vorgesehen sein, daß der Außenrohrschaft 24 relativ zu dem Schaft 14 bzw. zu dem Griff 12 verdrehbar ist, so daß beim Ein- bzw. Ausdrehen der Schraube 120 der Außenrohrschaft 24 nicht mitgedreht wird.

## Patentansprüche

1. Schraubendreher zum Einbringen und Eindrehen einer Schraube (120) im menschlichen oder tierischen Körper und/oder zum Ausdrehen und Entnehmen einer Schraube aus dem menschlichen oder tierischen Körper, mit einem Griff (12) und mit einem Schaft (14), dessen vorderes Ende einen mit einem Schraubenkopf (124) der Schraube (120) zum Ein- bzw. Ausdrehen der Schraube (120) in Eingriff bringbaren Kopf (16) aufweist, sowie mit einer Festhalteeinrichtung (18) zum Festhalten der Schraube (120) an dem Schaft (14), wobei die Festhalteeinrichtung (18) einen am vorderen Ende eine in ihre Offenlage vorgespannte Spannzange (20) aufweisenden, den Schaft (14) umgebenden Innenrohrschaft (22) und einen den Innenrohrschaft (22) umgebenden Außenrohrschaft (24) aufweist, und mit einer Betätigungseinrichtung (26) für den Innenrohrschaft (22) und den Außenrohrschaft (24), die von einer ersten Stellung in eine zweite Stellung bewegbar ist, wodurch die Spannzange (20) aus einer zurückgezogenen Stellung über den Kopf (16) verschoben wird, und wobei die Betätigungseinrichtung (26) aus der zweiten Stellung in eine dritte Stellung bewegbar ist, wodurch der Außenrohrschaft (24) relativ zu dem Innenrohrschaft (22) über die Spannzange (20) verschoben wird, um diese zu schließen, und umgekehrt, wobei der Innenrohrschaft (22) und/oder der Außenrohrschaft (24) abnehmbar mit dem Griff (12) verbunden ist/sind, **dadurch gekennzeichnet, daß** die Betätigungseinrichtung (26) am Griff (12) angeordnet ist und in eine vierte Stellung bewegbar ist, in der der Innenrohrschaft (22) und/oder der Außenrohrschaft (24) von dem Griff (12) abnehmbar bzw. an diesen anbringbar ist/sind, wobei der Innenrohrschaft (22) und/oder der Außenrohrschaft (24) durch Bewegen der Betätigungseinrichtung (26) von der vierten Stellung in die erste Stellung an dem Griff (12) arretiert bzw. umgekehrt entarretiert wird/werden.

2. Schraubendreher nach Anspruch 1, **dadurch gekennzeichnet, daß** der Innenrohrschaft (22) und der Außenrohrschaft (24) durch Bewegung der Betätigungseinrichtung (26) zwischen der ersten und der zweiten Stellung gemeinsam axial verschoben werden.

3. Schraubendreher nach einem der Ansprüche 1 bis 2, **dadurch gekennzeichnet, daß** der Innenrohrschaft (22) und/oder der Außenrohrschaft (24) durch eine Schnellöseverbindung mit dem Griff (12) verbunden ist/sind.

4. Schraubendreher nach einem der Ansprüche 1 bis 3, **dadurch gekennzeichnet, daß** die Betätigungseinrichtung (26) nur nach Entriegeln einer Sperre in die vierte Stellung bewegbar ist.

5. Schraubendreher nach Anspruch 4, **dadurch gekennzeichnet, daß** die Sperre mittels eines Druckknopfes entriegelbar ist.

6. Schraubendreher nach Anspruch 4, **dadurch gekennzeichnet, daß** die Sperre durch Verdrehen der Betätigungseinrichtung (26) entriegelbar ist.

7. Schraubendreher nach einem der Ansprüche 1 bis 6, **dadurch gekennzeichnet, daß** die Betätigungseinrichtung (26) in der ersten, zweiten, dritten und/oder der vierten Stellung einrastet.

8. Schraubendreher nach Anspruch 7, **dadurch gekennzeichnet, daß** die Betätigungseinrichtung (26) für die erste, zweite, dritte und/oder vierte Stellung Kugelrasten (68, 72) aufweist.

9. Schraubendreher nach einem der Ansprüche 1 bis 8, **dadurch gekennzeichnet, daß** die Betätigungseinrichtung (26) zumindest einen Schieber (48) aufweist, der am Griff (12) axial verschiebbar angeordnet ist.

10. Schraubendreher nach Anspruch 9, **dadurch gekennzeichnet, daß** der Schieber (48) am vorderen Ende des Griffes (12) angeordnet ist.

11. Schraubendreher nach Anspruch 9 oder 10, **dadurch gekennzeichnet, daß** die Betätigungseinrichtung (26) zumindest ein erstes Steuerelement (74) aufweist, das auf seiner Innenseite (94) eine als Steuerkurve ausgebildete Nut (96)aufweist, in die ein am hinteren Ende des Außenrohrschaftes (24) angeordneter Stift (38) eingreift, wobei das Steuerelement (74) um eine quer zur Längsrichtung des Schafts (14) verlaufende Schwenkachse (78)verschwenkbar am Griff (12) gelagert ist, und wobei der Schieber (48) einen Stift (90) aufweist, der in eine Führungsnut (88) auf einer Außenseite (86) des Steuerelementes (74) eingreift, so daß durch axiales Verschieben des Schiebers (48) das Steuerelement (74) verschwenkt und dadurch der Außenrohrschaft (24) verschoben wird.

12. Schraubendreher nach einem der Ansprüche 9 bis 11, **dadurch gekennzeichnet, daß** die Betätigungseinrichtung (26) zumindest ein zweites Steuerelement (76) aufweist, das auf seiner Innenseite (98) eine als Steuerkurve ausgebildete Nut (100) aufweist, in die ein am hinteren Ende des Innenrohrschaftes (22) angeordneter Stift (34) eingreift, wobei das Steuerelement (76) um eine quer zur Längsrichtung des Schafts verlaufende Schwenkachse (78) verschwenkbar am Griff (12) gelagert ist, und wobei der Schieber (48) einen Stift (92) aufweist, der in eine Führungsnut auf der Außenseite des Steuerelementes (76) eingreift, so daß durch axiales Verschieben des Schiebers (48) das Steuerelement (76) verschwenkt und dadurch der Innenrohrschaft (22) verschoben wird.

13. Schraubendreher nach einem der Ansprüche 11 oder 12, **dadurch gekennzeichnet, daß** die Nut (96) des ersten Steuerelementes (74) bzw. die Nut (100) des zweiten Steuerelementes (76) an ihrem vorderseitigen Ende offen ist, und daß der Stift (38) des Außenrohrschaftes (24) bzw. der Stift (34) des Innenrohrschaftes (22) axial in die Nut (96, 100) einfahren und aus dieser ausfahren kann, wenn die Betätigungseinrichtung (26) in ihrer vierten Stellung ist, und daß der Stift (34) des Innenrohrschaftes (22) bzw. der Stift (38) des Außenrohrschaftes (24) in der Nut (96, 100) gefangen ist, sobald die Betätigungseinrichtung (26) in die erste Stellung bewegt worden ist.

14. Schraubendreher nach einem der Ansprüche 10 bis 13, **dadurch gekennzeichnet, daß** eine distale Stirnseite (106) des Schiebers (48) eine nicht runde Öffnung aufweist, durch die der Außenrohrschaft (24) durchgeführt ist, wobei ein hinterer Endabschnitt (36) des Außenrohrschaftes (24) eine entsprechende komplementäre Umfangsgestalt aufweist.

15. Schraubendreher nach einem der Ansprüche 1 bis 9, **dadurch gekennzeichnet, daß** die Betätigungseinrichtung (26) zwei Schieber aufweist, die am Griff axial verschiebbar angeordnet sind, wobei der eine Schieber mit dem Außenrohrschaft und der andere Schieber mit dem Innenrohrschaft verbunden ist.

16. Schraubendreher nach Anspruch 15, **dadurch gekennzeichnet, daß** der eine Schieber zwischen der ersten und der zweiten Stellung bewegbar ist, um die geöffnete Spannzange axial zu verschieben, und daß der andere Schieber zwischen der zweiten Stellung und der dritten Stellung bewegbar ist, um den Außenrohrschaft relativ zu dem Innenrohrschaft zu verschieben, um die Spannzange zu schließen bzw. zu öffnen.

17. Schraubendreher nach einem der Ansprüche 1 bis 9, **dadurch gekennzeichnet, daß** die Betätigungseinrichtung (26) ein am Griff angeordetes drehbares Griffelement aufweist, das in die erste, zweite, dritte und vierte Stellung gedreht werden kann.

18. Schraubendreher nach Anspruch 17, **dadurch gekennzeichnet, daß** das drehbare Griffelement innenliegende Steuerkurven aufweist, in die am Außenrohrschaft und am Innenrohrschaft vorgesehene Führungsstifte eingreifen, wobei die Steuerkurven derart ausgebildet sind, daß eine Drehung des Griffelementes in eine axiale Bewegung des Innenrohrschaftes und des Außenrohrschaftes sowie eine Relativverschiebung zwischen dem Innenrohrschaft und dem Außenrohrschaft übertragen wird.

19. Schraubendreher nach einem der Ansprüche 1 bis 18, **dadurch gekennzeichnet, daß** der Innenrohrschaft zumindest in der dritten Stellung der Betätigungseinrichtung (26) relativ zu dem Außenrohrschaft (24) verdrehbar ist.

20. Schraubendreher nach einem der Ansprüche 1 bis 19, **dadurch gekennzeichnet, daß** der Kopf des Schafts auswechselbar ist.

## Claims

1. A screwdriver for introducing and screwing a screw (120) in the human or animal body and/or for unscrewing and removing a screw from the human or animal body, comprising a handle (12) and a shaft (14) whose front end has a head (16) that can be brought into engagement with a screw head (124) of the screw (120) for screwing the screw (12) in and out, and comprising a retention device (18) for retaining the screw (120) on the shaft (14), the retention device (18) having an inner tubular shaft (22) which surrounds the shaft (14) and has at the front end a clamping gripper (20) preloaded into its open position, and an outer tubular shaft (24) surrounding the inner tubular shaft (22), and comprising an actuation device (26) for the inner tubular shaft (22) and outer tubular shaft (24) that is movable from a first position into a second position, as a result of which the clamping gripper (20) is displaced over the head (16) from a pulled-back position, and the actuation device (26) being movable from the second position into a third position, as a result of which the outer tubular shaft (24) is displaced relative to the inner tubular shaft (22) over the clamping gripper (20) in order to close the latter, and vice versa, the inner tubular shaft (22) and/or outer tubular shaft (24) being joined removably to the handle (12), **characterized in that**
the actuation device (26) is arranged on the handle (12) and is movable into a fourth position in which the inner tubular shaft (22) and/or the outer tubular shaft (24) is/are removable from or mountable on the handle (12), the inner tubular shaft (22) and/or the outer tubular shaft (24) being locked on the handle (12) by moving the actuation device (26) from the fourth position into the first position, or are, vice versa, unlocked.

2. The screwdriver of claim 1, **characterized in that** the inner tubular shaft (22) and the outer tubular shaft (24) are together axially displaced by moving the actuation device (26) between the first and the second position.

3. The screwdriver of anyone of claims 1 through 2, **characterized in that** the inner tubular shaft (22) and/or the outer tubular shaft (24) is/are joined to the handle (12) by a quick-release connection.

4. The screwdriver of anyone of claims 1 through 3, **characterized in that** the actuation device (24) is movable into the fourth position only after an interlock has been disengaged.

5. The screwdriver of claim 4, **characterized in that** the interlock can be disengaged by way of a pushbutton.

6. The screwdriver of claim 4, **characterized in that** the interlock can be disengaged by rotating the actuation device (26).

7. The screwdriver of anyone of claims 1 through 6, **characterized in that** the actuation device (26) snaps into the first, second, third, and/or fourth position.

8. The screwdriver of claim 7, **characterized in that** the actuation device (26) has ball catches (68, 72) for the first, second, third, and/or fourth position.

9. The screwdriver of anyone of claims 1 through 8, **characterized in that** the actuation device (26) has at least one slider (48) that is arranged in axially displaceable fashion on the handle (12).

10. The screwdriver of claim 9, **characterized in that** the slider (48) is arranged at the front end of the handle (12).

11. The screwdriver of claim 9 or 10, **characterized in that** the actuation device (26) has at least one first control element (74) that has on its inner side (94) a groove (96) configured as a cam curve into which a pin (38) arranged at the rear end of the outer tubular shaft (24) engages, the control element (74) being mounted on the handle (12) pivotably about a pivot axis (78) running transversely to the longitudinal direction of the shaft (14), and the slider (48) having a pin (90) that engages into a guide groove (88) on an outer side (86) of the control element (74), so that by axial displacement of the slider (48), the control element (74) is pivoted and the outer tubular shaft (24) is thereby displaced.

12. The screwdriver of anyone of claims 9 through 11, **characterized in that** the actuation device (26) has at least one second control element (76) that has on its inner side (98) a groove (100), configured as a cam curve, into which engages a pin (34) arranged at the rear end of the inner tubular shaft (22), the control element (76) being mounted on the handle (12) pivotably about a pivot axis (78) running transversely to the longitudinal direction of the shaft, and the slider (48) having a pin (92) which engages into a guide groove on the outer side of the control element (76) so that by axial displacement of the slider (48), the control element (76) is pivoted and the inner tubular shaft (22) is thereby displaced.

13. The screwdriver of claim 11 or 12, **characterized in that** the groove (96) of the first control element (74) and the groove (100) of the second control element (76) are open at their front ends; and the pin (38) of the outer tubular shaft (24) or the pin (34) of the inner tubular shaft (22) can enter the groove (96, 100) and emerge from it axially when the actuation device (26) is in its fourth position; and the pin (34) of the inner tubular shaft (22) and the pin (38) of the outer tubular shaft (24) are captured in the groove (96, 100) as soon as the actuation device (26) has been moved into the first position.

14. The screwdriver of anyone of claims 10 through 13, **characterized in that** one distal end face (106) of the slider (48) has a non-round opening through which the outer tubular shaft (24) is passed, a rear end segment (36) of the outer tubular shaft (24) having a corresponding complementary peripheral shape.

15. The screwdriver of anyone of claims 1 through 9, **characterized in that** the actuation device (26) has two sliders that are arranged in axially displaceable fashion on the handle, the one slider being joined to the outer tubular shaft and the other slider to the inner tubular shaft.

16. The screwdriver of claim 15, **characterized in that** the one slider is movable between the first and the second position in order to axially displace the open clamping gripper; and the other slider is movable between the second and the third position in order to displace the outer tubular shaft relative to the inner tubular shaft in order to close or open the clamping gripper.

17. The screwdriver of anyone of claims 1 through 9, **characterized in that** the actuation device (26) has a rotatable handle element, arranged on the handle, that can be rotated into the first, second, third, and fourth position.

18. The screwdriver of claim 17, **characterized in that** the rotatable handle element has internally located cam curves into which guide pins provided on the outer tubular shaft and inner tubular shaft engage, the cam curves being configured such that a rotation of the handle element is converted into an axial movement of the inner tubular shaft and outer tubular shaft, and a relative displacement between the inner tubular shaft and outer tubular shaft.

19. The screwdriver of anyone of claims 1 through 18, **characterized in that** the inner tubular shaft is, at least in the third position of the actuation device (26), rotatable relative to the outer tubular shaft (24).

20. The screwdriver of anyone of claims 1 through 19, **characterized in that** the head of the shaft is replaceable.

## Revendications

1. Tournevis pour introduire et visser une vis (120) dans un corps humain ou animal et/ou pour dévisser et enlever une vis du corps humain ou animal, comportant une poignée (12) et une tige (14) dont l'extrémité avant comporte une tête (16) pouvant être amenée en prise avec une tête de vis (124) de la vis(120) pour visser et dévisser la vis (120), ainsi qu'un dispositif d'immobilisation (18) pour immobiliser la vis (120) sur la tige (14), le dispositif d'immobilisation (18) comportant une tige tubulaire intérieure (22) entourant la tige (14) et présentant à son extrémité avant une pince de serrage (20) précontrainte dans sa position ouverte, ainsi qu'une tige tubulaire extérieure (24) entourant la tige tubulaire intérieure (22), et comportant un dispositif d'actionnement (26) pour la tige tubulaire intérieure (22) et la tige tubulaire extérieure (24), lequel est déplaçable d'une première position dans une deuxième position, ce qui fait que la pince de serrage (20) est déplacée d'une position en retrait sur la tête (16), et le dispositif d'actionnement (26) étant déplaçable de la deuxième position dans une troisième position, ce qui fait que la tige tubulaire extérieure (24) est déplacée par rapport à la tige intérieure tubulaire (22) sur la pince de serrage (20), pour fermer celle-ci, et inversement, la tige tubulaire intérieure (22) et/ou la tige tubulaire extérieure (24) étant reliée(s) de manière séparable à la poignée (12), **caractérisé en ce que** le dispositif d'actionnement (26) est disposé sur la poignée (12) et est déplaçable dans une quatrième position dans laquelle la tige tubulaire intérieure (22) et/ou la tige tubulaire extérieure (24) peuvent être enlevées de la poignée (12) ou placées sur celle-ci, la tige tubulaire intérieure (22) et/ou la tige tubulaire extérieure (24) étant bloquée(s) ou à l'inverse débloquée(s) sur la poignée (12) par déplacement du dispositif d'actionnement (26) de la quatrième position dans la première position.

2. Tournevis selon la revendication 1, **caractérisé en ce que** la tige tubulaire intérieure (22) et la tige tubulaire extérieure (24) sont déplacées axialement ensemble par déplacement du dispositif d'actionnement (26) entre la première et la deuxième position.

3. Tournevis selon l'une des revendications 1 ou 2, **caractérisé en ce que** la tige tubulaire intérieure (22) et/ou la tige tubulaire extérieure (24) est/sont reliée(s) à la poignée (12) par une liaison rapide.

4. Tournevis selon l'une des revendications 1 à 3, **caractérisé en ce que** le dispositif d'actionnement (26) n'est déplaçable dans la quatrième position qu'après déverrouillage d'un élément de blocage.

5. Tournevis selon la revendication 4, **caractérisé en ce que** l'élément de blocage peut être déverrouillé au moyen d'un bouton-poussoir.

6. Tournevis selon la revendication 4, **caractérisé en ce que** l'élément de blocage peut être déverrouillé par rotation du dispositif d'actionnement (26).

7. Tournevis selon l'une des revendications 1 à 6, **caractérisé en ce que** le dispositif d'actionnement (26) s'indexe dans la première, la deuxième, la troisième et/ou la quatrième position.

8. Tournevis selon la revendication 7, **caractérisé en ce que** le dispositif d'actionnement (26) comporte des organes d'indexage à billes (68, 72) pour la première, la deuxième, la troisième et/ou la quatrième position.

9. Tournevis selon l'une des revendications 1 à 8, **caractérisé en ce que** le dispositif d'actionnement (26) comporte au moins un coulisseau (48) qui est disposé sur la poignée (12) de manière à pouvoir coulisser axialement.

10. Tournevis selon la revendication 9, **caractérisé en ce que** le coulisseau (48) est disposé à l'extrémité avant de la poignée (12).

11. Tournevis selon la revendication 9 ou 10, **caractérisé en ce que** le dispositif d'actionnement (26) comporte au moins un premier élément de commande (74) qui présente, sur son côté intérieur (94), une rainure (96) conformée en came de commande, dans laquelle s'engage un doigt (38) disposé à l'extrémité arrière de la tige tubulaire extérieure (24), l'élément de commande (74) étant monté sur la poignée (12) de manière à pouvoir pivoter autour d'un axe de pivotement (78) s'étendant transversalement à la direction longitudinale de la tige (14), et le coulisseau (48) comportant un doigt (90) qui s'engage dans une rainure de guidage (88) sur un côté extérieur (86) de l'élément de commande (74), de manière que par coulissement axial du coulisseau (48), l'élément de commande (74) pivote et que par là la tige tubulaire extérieure (24) soit déplacée.

12. Tournevis selon l'une des revendications 9 à 11, **caractérisé en ce que** le dispositif d'actionnement (26) comporte au moins un deuxième élément de commande (76) qui présente, sur son côté intérieur (98), une rainure (100) conformée en came de commande dans laquelle s'engage un doigt (34), disposé à l'extrémité arrière de la tige tubulaire intérieure (22), l'élément de commande (76) étant monté sur la poignée (12) de manière à pouvoir pivoter sur un axe de pivotement (78) s'étendant transversalement à la direction longitudinale de la tige, et le coulisseau (48) comportant un doigt (92) qui s'engage dans une rainure de guidage sur le côté extérieur de l'élément de commande (76) de manière que par coulissement axial du coulisseau (48), l'élément de commande (76) pivote et que par là la tige tubulaire intérieure (22) soit déplacée.

13. Tournevis selon l'une des revendications 11 ou 12, **caractérisé en ce que** la rainure (96) du premier élément de commande (14) ou la rainure (100) du deuxième élément de commande (76) est ouverte à son extrémité côté avant, et **en ce que** le doigt (38) de la tige tubulaire extérieure (24) ou le doigt (34) de la tige tubulaire intérieure (22) peut s'introduire axialement dans la rainure (96, 100) ou ressortir de celle-ci lorsque le dispositif d'actionnement (26) se trouve dans sa quatrième position, et **en ce que** le doigt (34) de la tige tubulaire intérieure (22) ou le doigt (38) de la tige tubulaire extérieure (24) est retenu dans la rainure (96, 100), dès que le dispositif d'actionnement (26) a été déplacé dans la première position.

14. Tournevis selon l'une des revendications 10 à 13, **caractérisé en ce qu'**une face frontale distale (106) du coulisseau (48) présente une ouverture non circulaire à travers laquelle est guidée la tige tubulaire extérieure (24), un tronçon terminal arrière (36) de la tige tubulaire extérieure (24) présentant une forme périphérique complémentaire correspondante.

15. Tournevis selon l'une des revendications 1 à 9, **caractérisé en ce que** le dispositif d'actionnement (26) comporte deux coulisseaux qui sont disposés sur la poignée de manière à pouvoir coulisser axialement, un coulisseau étant relié à la tige tubulaire extérieure et l'autre coulisseau à la tige tubulaire intérieure.

16. Tournevis selon la revendication 15, **caractérisé en ce qu'**un coulisseau est déplaçable entre la première et la deuxième position, afin de faire coulisser axialement la pince de serrage ouverte, et **en ce que** l'autre coulisseau est déplaçable entre la deuxième position et la troisième position, afin de faire coulisser la tige tubulaire extérieure par rapport à la tige tubulaire intérieure, pour fermer ou ouvrir la pince de serrage.

17. Tournevis selon l'une des revendications 1 à 9, **caractérisé en ce que** le dispositif d'actionnement (26) comporte un élément de préhension tournant, disposé sur la poignée, et qui peut être tourné dans la première, la deuxième, la troisième et la quatrième position.

18. Tournevis selon la revendication 17, **caractérisé en ce que** l'élément de préhension tournant comporte des cames de commande situées à l'intérieur dans lesquelles s'engagent des doigts de guidage prévus sur la tige tubulaire extérieure et la tige tubulaire intérieure, les cames de commande étant conformées de manière à transformer une rotation de l'élément de préhension en un mouvement axial de la tige tubulaire intérieure et de la tige tubulaire extérieure, ainsi qu'en un coulissement relatif entre la tige tubulaire intérieure et la tige tubulaire extérieure.

19. Tournevis selon l'une des revendications 1 à 18, **caractérisé en ce que** la tige tubulaire intérieure peut tourner par rapport à la tige tubulaire extérieure (24), au moins dans la troisième position du dispositif d'actionnement (26).

20. Tournevis selon l'une des revendications 1 à 19, **caractérisé en ce que** la tête de la tige est échangeable.
